# EUROPEAN PATENT APPLICATION

(11) **EP 4 570 207 A1**
(43) Date of publication of application: **18.06.2025**
(21) Application number: 24218923.1
(22) Date of filing: 11.12.2024
(51) Int. Cl.: A61B 18/14

(54) **LOCAL DETECTION OF CATHETER TO TISSUE PROXIMITY WITH ENHANCED SPATIAL COVERAGE**

(30) Priority: 12.12.2023 US 202318536392
(71) Applicant: Biosense Webster (Israel) Ltd., 2066717 Yokneam (IL)
(72) Inventor: GOVARI, Assaf, 2066717 Yokneam (IL); GLINER, Vadim, 2066717 Yokneam (IL); ALTMANN, Andres Claudio, 2066717 Yokneam (IL)
(74) Representative: Carpmaels & Ransford LLP

(57) **Abstract**

A system includes a catheter, a signal generator, an interface, and a processor. The catheter includes an expandable distal-end assembly including (i) a plurality of functional electrodes that are at least partially external to an inner volume of the assembly, the functional electrodes configured to be placed in contact with wall tissue of a cardiac chamber, (ii) a proximal reference electrode located at a proximal end of the assembly externally to the inner volume, and (iii) a distal reference electrode located at a distal edge of the expandable assembly externally to the inner volume. The signal generator is configured to generate the AC signal between the proximal and distal reference electrodes. The interface is configured to sense the resulting electrical AC signal. The processor is configured to, based on the sensed AC signals, determine, for at least one given functional electrode, a proximity of the electrode to the wall tissue.

## Description

### FIELD OF THE DISCLOSURE

The present disclosure relates generally to invasive medical probes, and particularly to estimation of proximity of catheters to tissue.

### BACKGROUND OF THE DISCLOSURE

Techniques to estimate the proximity of an electrode of a catheter to tissue were previously proposed in the patent literature. For example, U.S. Patent Application Publication 2021/0059743 describes a system including an expandable frame and a processor. The expandable frame includes (i) one or more ablation electrodes disposed over an external surface of the frame and configured to be placed in contact with wall tissue of a cavity of a patient, and (ii) stem and edge electrodes coupled just proximally and just distally to the balloon, respectively. The processor is configured to: (a) measure one or more first impedances between one or more of the ablation electrodes and the stem electrode, (b) measure one or more second impedances between one or more of the ablation electrodes and the edge electrode, and (c) based on the first and second impedances, determine, for at least an ablation electrode from among the one or more ablation electrodes, whether the ablation electrode is in physical contact with the wall tissue.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electro-anatomical (EA) mapping and ablation system, in accordance with an example of the present disclosure;
Fig. 2 is a schematic, pictorial illustration of a basket assembly configured to electrically sense proximity of a functional electrode to cavity wall tissue, in accordance with an example of the present disclosure; and
Fig. 3 is a schematic, pictorial illustration of the basket assembly of Fig. 2 with some of its functional electrodes located deep in the blood pool while others in proximity to wall tissue, in accordance with an example of the present disclosure.
Fig. 4 is a flow chart that schematically illustrates a method and algorithm to estimate the proximity of a functional electrode to cavity wall tissue, in accordance with an example of the present disclosure.

### DETAILED DESCRIPTION OF EXAMPLES

### OVERVIEW

Wall tissue of a cardiac chamber can be electro-anatomically mapped and/or ablated using a catheter having multiple functional electrodes fitted at an expandable distal-end assembly of the catheter. In the mapping and/or ablation procedure in a cardiac chamber, the physician manipulates the expanded distal-end assembly for the electrodes to contact chamber walls to acquire and/or apply electrical signals.

The quality of electrical mapping and/or ablation depends on the proximity of the functional electrodes to the cavity wall tissue. The proximity may be evaluated using a parameter typically referred to as tissue proximity indication (TPI)." A proximity scale may use arbitrary units (e.g., an index ranging from 1 to 10) or can be given in physical units of distance.

For example, TPI can be estimated from AC impedance readings by measuring the electrical impedance between functional electrodes in unknown proximity with tissue, that are disposed over the expandable distal assembly and a reference ring electrode located over a proximal base section of the expandable assembly, the reference electrode is located externally to an inner volume defined by the expandable assembly. Examples of such an electrical technique are described in the U.S. Patent Application 18/530,268 field December 6, 2023, and titled, "Reference Electrode Dedicated to Catheter Tissue Proximity Estimation."

However, for a large distal end assembly, electrical measurements for the more distal electrodes may be difficult (e.g., as the impedances relative to the reference electrode on the base section may not be sensitive enough to tissue proximity).

Examples of the present disclosure that are described herein enhance the coverage of proximity sensing by adding to the proximal ring electrode an effective distal reference electrode at the assembly's distal end, that is also located externally to an inner volume defined by the expandable assembly. During operation, an AC signal generator applies an AC signal between the distal and proximal ends to establish an electric field therebetween.

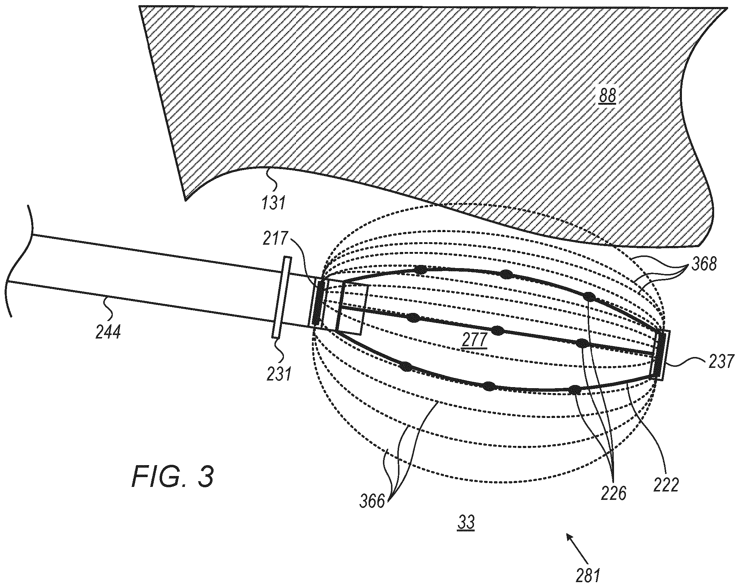

The electric field increases about some functional electrodes as the assembly is brought in proximity to wall tissue, due to an increase in electrical impedance via the tissue. The reason for the increase is that the electric field lines are pushed away from tissue and become denser at the adjacent blood, as seen in Fig. 3. Typically, both the distal and proximal reference electrodes remain in the blood pool.

A circuitry is used to measure a resulting AC signal at each of the functional electrodes to detect the increased impedance. In case the field is applied using a constant magnitude current source, the functional electrodes act like independent voltage sensors. The voltage increases with impedance. If a voltage source is used, the functional electrodes act like independent current sensors. The current decreases with impedance.

Resulting changes in AC voltage and/or current signals are sensed between a functional electrode and another given electrode such as the proximal reference ring electrode and/or the distal reference electrode, and/or a far-field electrode located within the inner volume and/or another functional electrode on the spline.

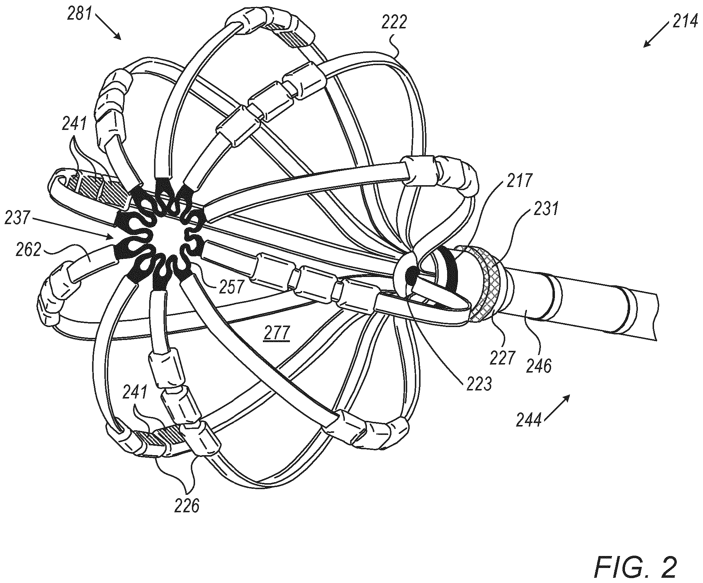

In some examples, such as in the example of a basket assembly of Fig. 2, the distal reference electrode is created by electrically connecting to the distal structure that connects the splines of the assembly at the distal end. In other examples, the distal electrode is disposed (e.g., on a membrane).

The change in impedances as function of the functional electrode's proximity to tissue for the detection of catheter proximity to tissue has enhanced spatial coverage, as described in Fig. 3.

### SYSTEM DESCRIPTION

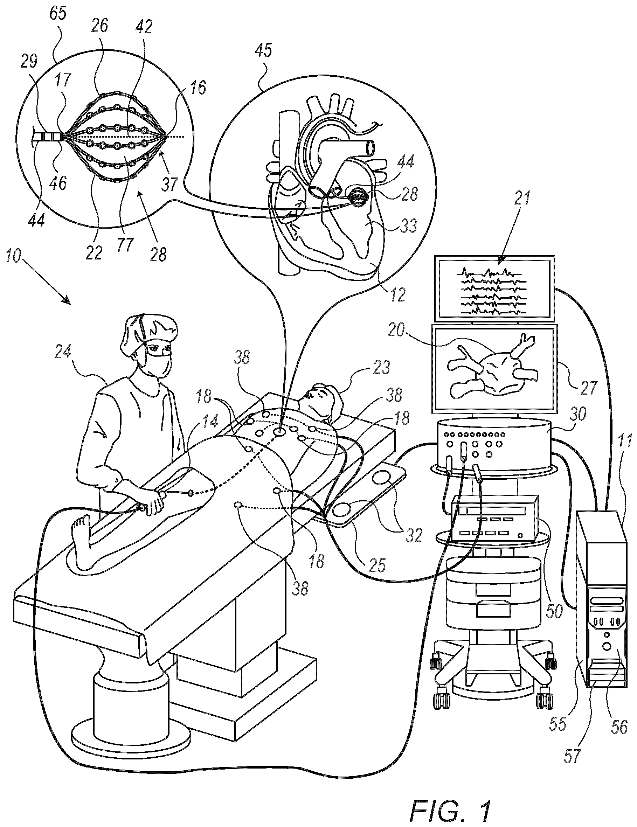

Fig. 1 is a schematic, pictorial illustration of a catheter-based electroanatomical (EA) mapping and ablation system 10, in accordance with an example of the present disclosure. System 20 is configured to determine, e.g., prior to performing diagnostics and/or ablation, whether a given functional electrode 26 of a plurality of functional electrodes 26 of a basket catheter 14 is having sufficient contact with (or proximity to) tissue or is immersed in blood pool 33 of a cardiac chamber.

System 10 includes one or more catheters, which are percutaneously inserted by physician 24 through the patient's vascular system into a chamber or vascular structure of a heart 12. Typically, a delivery sheath catheter is inserted into the left or right atrium near a desired location in heart 12. Thereafter, one or more catheters in turn can be inserted into the delivery sheath catheter to arrive at the desired location. The one or more catheters may include catheters dedicated for sensing intracardiac electrogram (IEGM) signals, catheters dedicated to ablating and/or catheters dedicated to both sensing and ablating. An example basket catheter 14 that is configured for sensing IEGM is illustrated herein. As seen in inset 45, physician 24 brings a basket type of expandable distal-end assembly 28 (also called hereinafter "expandable distal-end assembly 28") fitted on a shaft 44 of catheter 14 into contact with the heart wall for sensing a target site in heart 12. For ablation, physician 24 similarly brings the distal end of an ablation catheter to a target site for ablating.

As seen in inset 65, catheter 14 is an exemplary catheter that includes one, and preferably multiple, functional electrodes 26 optionally distributed over a plurality of splines 22 at expandable distal-end assembly 28 and configured to sense IEGM signals. Catheter 14 additionally includes a proximal position sensor 29 (e.g., TAS 29 comprising three EMCs) embedded in a distal end 46 of shaft 44 near expandable distal end assembly 28, to track the position of the distal end of expandable distal end assembly 28. Optionally, and preferably, position sensor 29 is a magnetic-based position sensor that includes magnetic coils for sensing three-dimensional (3D) position. Distal end 46 of shaft 44 may comprise an amplifying circuit configured to amplify the output from the three EMCs of sensor 29.

Magnetic position sensor 29 is operated together with an external location pad 25 that includes a plurality of magnetic coils 32 configured to generate magnetic fields in a predefined working volume. Using the operation with external location pad 25 (at a different frequency for each EMC), the processor can determine the position of EMCs 29 on a coordinate system of the position tracking system.

Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of functional electrodes 26. For impedance-based tracking, electrical current is directed toward electrodes 26 and sensed at electrode skin patches 38, such that the location of each electrode can be triangulated via electrode patches 38. Real-time orientation of expandable distal end assembly 28 of catheter 14 can be calculated from tracked locations of electrodes 26. This relative orientation is manifested by an angle formed between distal end 46 and a longitudinal axis 42 of expandable assembly 28 (to a distal edge 16 of the assembly).

Details of the impedance-based location tracking technology are described in US Patent Nos. 7,536,218; 7,756,576; 7,848,787; 7,869,865; and 8,456,182.

Catheter 14 is configured to acquire electrical signals indicative of proximity of any given functional electrode 26 to the wall tissue of heart 12. To this end, a signal generator 35 is configured to generate an AC signal between a distal reference electrode 37 and a proximal reference ring electrode 17 located both externally to an inner volume 77 defined by the splines of assembly 28.

Signal generator 35 may be a voltage source and/or a current source or a combination of both. A readout circuitry in PIU 30 is configured to monitor (e.g., sense) respectively resulting AC signal between each of functional electrodes 26 and a reference electrode, such as the distal reference electrode 37 and/or the proximal reference electrode 17. The sensed AC signal is used for estimating each functional electrode's 26 proximity to tissue, as described below in Fig. 3.

A recorder 11 displays electrograms 21 captured with body surface ECG electrodes 18 and intracardiac electrograms (IEGM) captured with functional electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to a subset of the plurality of electrodes 26 at the distal assembly 28 of catheter 14 configured for ablating. Energy produced by ablation energy generator 50 may include but is not limited to, radiofrequency (RF) energy or pulsed-field ablation (PFA) energy, including monopolar or bipolar high-voltage DC pulses that may be used to effect irreversible electroporation (IRE), or combinations thereof.

Patient interface unit (PIU) 30 is configured to establish electrical communication between catheters, electrophysiological equipment, power supply and a workstation 55 for controlling the operation of system 10. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, processor unit 56 with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of system 10 is available as the CARTO^{™} 3 System, available from Biosense Webster, Inc., 31A Technology Drive, Irvine, CA 92618.

While Fig. 1 describes a basket assembly, the disclosed technique can be applied, *mutatis mutandis,* to an expandable balloon assembly having an expandable membrane, wherein the functional electrodes are disposed over the membrane.

### ESTIMATION OF TOUCH PROXIMITY (TP) OF A FUNCTIONAL ELECTRODE OF EXPANDABLE ASSEMBLY

Fig. 2 is a schematic, pictorial illustration of a basket catheter assembly 281 configured for electrical measurement of the proximity of a functional electrode 226 to cavity wall tissue, in accordance with an example of the present disclosure. Basket assembly 281 can be used to implement basket assembly 28 of Fig. 1 above. As seen, assembly 281 is part of a catheter 214 further comprising a shaft 244 having a distal end 246. Distal-end assembly 228 includes a proximal base 227 configured to couple the assembly to a distal end 246 of the shaft 244.

Basket assembly 281 is realized as an expandable frame comprising multiple splines 222, wherein the functional electrodes 226 are coupled to the splines. Splines 222 are each electrically insulated from the environment over most of their area by an insulation layer 262.

When expanded, as shown in Fig. 2, the expandable distal-end assembly 281 defines an inner volume 277. At the base of the assembly, inside volume 277, a far-field electrode 223 is used to remove far-field signals from IEGM signals acquired by electrodes 226.

The plurality of functional electrodes 226 are at least partially external to the inner volume and are configured to be placed in contact with wall tissue of the cavity. A proximal reference ring electrode 217 (such as ring electrode 17 of Fig. 1) is located on the proximal base 227 of the expandable distal-end assembly 281 externally to the inner volume 277. The position of the ring-shaped electrode 217 over base 227 is set such to avoid contact with tissue wall while the distal end assembly 281 is in an expanded state.

A distal reference ring electrode 237 (such as ring electrode 37 of Fig. 1) is located on the distal edge of the expandable distal-end assembly 281 externally to the inner volume 277. The distal reference electrode 237 is realized by the electrically uninsulated distal portion 257 of a Nitinol assembly 281. By being centered over the distal edge, the distal reference electrode 237 is configured to be placed in the cavity while being prevented from contacting the wall tissue. The distal electrode 237 is electrically realized (e.g., serving as a pole) by wiring electrically splines 222 to a circuitry for applying AC signal. The other pole of such circuitry is the proximal reference electrode 217.

The proximal base section 227 further comprises a mechanical guard ring 231 that protrudes from the proximal base further than the reference electrode 227, to prevent the reference electrode 227 from contacting the wall tissue.

Processor 56 applies an AC signal between reference electrodes 237 and 217. The processor estimates the proximity of a given electrode 226 to wall tissue (wall tissue not shown) based on monitored resulting AC signals between electrodes 226 and reference electrodes 237 and 217. The processor may use the monitored AC signals (e.g., voltages or currents) or changes in monitored AC signals relative to baseline AC signals obtained when all electrodes are deep inside a pool of blood, as detailed in Fig. 3.

Further improvement of the measurement accuracy can be achieved by applying inner electrical insulation coating 241 to electrode 226 so that the electrode portion in the blood is minimized upon contact with tissue. Coting 241 may be a polymer type or another dielectric layer (e.g., Silicon Nitride).

While Fig. 2 describes a basket assembly, the disclosed technique can be applied, *mutatis mutandis,* to an expandable balloon assembly having an expandable membrane, wherein the functional electrodes and the distal reference electrode are disposed over the membrane.

### ESTIMATION OF TOUCH PROXIMITY (TP) OF A DISTAL FUNCTIONAL ELECTRODE OF EXPANDABLE ASSEMBLY

Fig. 3 is a schematic, pictorial illustration of the basket assembly 281 of Fig. 2 with some of its functional electrodes 226 located deep in the blood pool 33 while others in proximity to wall 81 of tissue 131, in accordance with an example of the present disclosure.

During operation, an AC signal generator 35 applies an AC signal between the distal reference electrode 237 and the proximal reference electrode 217 to establish an electric field lines (366, 368) therebetween.

As seen, as assembly 281 is brought in proximity to wall tissue 131, the electric field lines 368 become denser about some functional electrodes 226, due to an increase in electrical impedance via tissue 88. The reason for the increase is that the electric field lines 368 are pushed away from tissue 88 and become denser at the adjacent blood. Typically, both the distal and proximal reference electrodes remain in the blood pool.

A circuitry (e.g., inside PIU 30) is used to measure a resulting AC signal at each of the functional electrodes 226 to detect the increased impedance. In case the field is applied using a constant magnitude current source, the functional electrodes 226 act like independent voltage sensors. The voltage increases with impedance. If a voltage source is used, the functional electrodes 226 act like independent current sensors. The current decreases with increased impedance.

The actual proximity of each functional electrode or of a subset (e.g., group) of electrodes can be presented to a user on a GUI in a form of the TPI scale.

### A METHOD FOR ESTIMATING OF TOUCH PROXIMITY (TP) OF A DISTAL FUNCTIONAL ELECTRODE OF EXPANDABLE ASSEMBLY

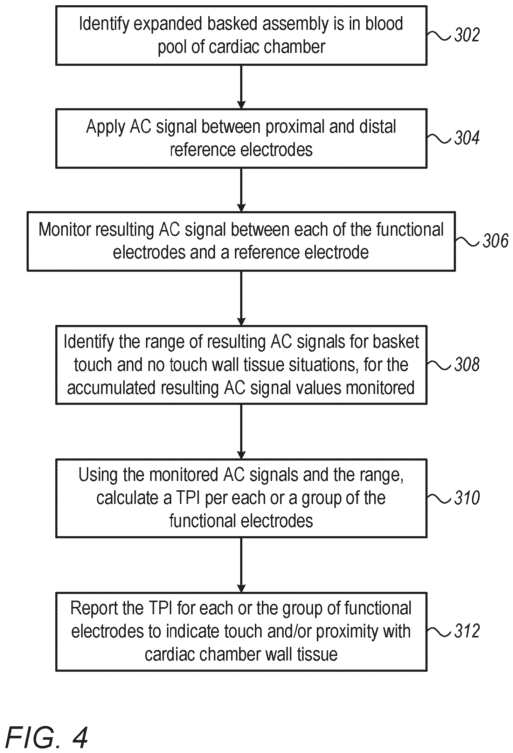

Fig. 4 is a flow chart that schematically illustrates a method and algorithm to estimate the proximity of a functional electrode to cavity wall tissue, in accordance with an example of the present disclosure. The algorithm, according to the present embodiment, carries out a process that begins with identifying that the expanded basket assembly 281 is in a blood pool 33 inside a cardiac cavity of heart 12, at a basket baseline positioning step 302. This identification can be done using fluoroscopy or contact force sensing to verify no mechanical interaction of basket assembly 281 with the cardiac chamber's wall tissue occurs at least in part of the time.

Next, at an AC signal (i.e., voltage or current) applying step 304, system 10 applies an AC signal between the reference electrodes 237 and 217.

Then, at resulting AC signals monitoring step 306, system 10 monitors resulting AC signals between each of functional electrodes 226 and a reference electrode, such as the distal reference electrode 237 and/or the proximal reference electrode 217 and/or far field electrode 223.

At resulting AC signal range identification step 308, as the basket occasionally contacts cavity wall tissue, the processor identifies the range of resulting AC signals for touch and no touch for the accumulated values monitored. The range can be determined from a conglomerate output from all the functional electrodes 226.

Using the monitored AC signals and the identified range of such, the processor calculates a TPI per each functional electrode or a group of such electrodes, at TPI calculation step 310.

Finally, at TPI reporting step 312, system 10 reports the TPI for each or a group of functional electrodes 226 to indicate touch and/or proximity with the cardiac chamber wall tissue of these functional electrodes.

The example flow chart shown in Fig. 4 is chosen purely for the sake of conceptual clarity. The present embodiment also comprises additional steps of the algorithm, such as acquiring intra-cardiac electrocardiograms, which have been omitted from the disclosure herein purposely to provide a more simplified flow chart. In addition, other steps, such as temperature measurements and applying irrigation, are omitted for clarity of presentation.

### EXAMPLES

### Example 1

A system (10) includes a catheter (14), a signal generator (35), an interface (30), and a processor (56). The catheter comprises a shaft (44) having a distal end (46) configured for insertion into a cardiac chamber of a patient (23), and an expandable distal-end assembly (28) that when expanded defines an inner volume (77), the distal-end assembly comprising (i) a plurality of functional electrodes (26) that are at least partially external to the inner volume (77) and are configured to be placed in contact with wall tissue (131) of the cardiac chamber, (ii) a proximal reference electrode (17) located at a proximal end of the expandable distal-end assembly (28) externally to the inner volume (77), (iii) a distal reference electrode (37) located at a distal edge (16) of the expandable distal-end assembly (28) externally to the inner volume (77). The proximal reference ring electrode (17) and the distal reference electrode (37) are configured to be coupled with one another and for, respectively, (i) generating an AC signal between the reference electrodes (17, 37), and (ii) sensing a resulting AC signal on each of the plurality of functional electrodes (26) and a given electrode (17, 37, 223). The signal generator (35) is configured to generate the AC signal between the distal reference electrode (37) and the proximal reference ring electrode (17). The interface (30) is configured to sense the resulting AC signal between each of the plurality of functional electrodes (26) and the given electrode (17, 37, 223). The processor (56) is configured to, based on the sensed AC signals, determine, for at least one given functional electrode (26) from among the plurality of functional electrodes, a proximity of the functional electrode (26) to wall tissue (131) of the cardiac chamber.

### Example 2

The system (10) according to example 1, wherein the given electrode (26) is one of the proximal reference ring electrode (17, 217), the distal reference electrode (37, 237), a far-field electrode (223) located within the inner volume (77, 277), and another functional electrode (26) on the spline.

### Example 3

The system (10) according to any of examples 1 and 2, wherein the signal generator (35) is configured to generate the AC signal by generating an AC voltage of constant magnitude, and wherein the interface (30) is configured to sense the respectively resulting AC signal by sensing a resulting AC current.

### Example 4

The system (10) according to any of examples 1 and 2, wherein the signal generator (35) is configured to generate the AC signal by generating an AC current of constant magnitude, and wherein the interface (30) is configured to sense the respectively resulting AC signal by sensing a resulting AC voltage.

### Example 5

The system (10) according to any of examples 1 through 4, wherein the proximal reference ring electrode (17, 217) is located on a proximal base (227) section of the expandable distal-end assembly (28, 281) externally to the inner volume (77, 277).

### Example 6

The system (10) according to any of examples 1 through 5, wherein the distal-end assembly (28, 281) is a basket having an expandable frame comprising multiple splines (22, 222) that are electrically insulated from a surrounding environment, wherein the functional electrodes (26, 226) are coupled to the splines (22, 222).

### Example 7

The system (10) according to any of examples 1 through 6, wherein the distal reference electrode (37, 237) is formed from distal ends of the splines (22, 222) that are (i) electrically exposed to the surrounding environment and (ii) electrically connected (257) to one another.

### Example 8

The system (10) according to example 1, wherein the distal-end assembly is a balloon assembly having an expandable membrane, and wherein the functional electrodes are disposed over the membrane.

### Example 9

The system (10) according to example 8, wherein the distal reference electrode is disposed over a distal end of the membrane.

### Example 10

A method includes inserting into a cardiac chamber of a patient a catheter (14) comprising a shaft (44, 244) having a distal end (46, 246), the catheter further comprising an expandable distal-end assembly (28, 281) that when expanded defines an inner volume (77, 277), the distal-end assembly comprising, (i) a plurality of functional electrodes (26) that are at least partially external to the inner volume and are configured to be placed in contact with wall tissue (131) of the cardiac chamber, (ii) a proximal reference electrode (17, 217) located at a proximal end of the expandable distal-end assembly (28, 281) externally to the inner volume, and (iii) a distal reference electrode (37, 237) located at a distal edge (16) of the expandable distal-end assembly externally to the inner volume, and wherein the proximal reference ring electrode and the distal reference electrode are to be coupled with one another and for, respectively, (i) generating an AC signal between the reference electrodes, and (ii) sensing a resulting AC signal on each of the plurality of functional electrodes and a given electrode (217, 237, 223). The AC signal is generated between the distal reference electrode (37, 237) and the proximal reference ring electrode (17, 217). The respectively resulting AC signal is sensed between each of the plurality of functional electrodes (26, 226) and the given electrode. Based on the sensed AC signals, it is determined, for at least one given functional electrode (26, 226) from among the plurality of functional electrodes, a proximity of the functional electrode to wall tissue (131) of the cardiac chamber.

### Example 11

A method, comprising:
inserting into a cardiac chamber of a patient a catheter comprising a shaft having a distal, the catheter further comprising an expandable distal-end assembly that when expanded defines an inner volume, the distal-end assembly comprising, (i) a plurality of functional electrodes that are at least partially external to the inner volume and are configured to be placed in contact with wall tissue of the cardiac chamber, (ii) a proximal reference electrode located at a proximal end of the expandable distal-end assembly externally to the inner volume, and (iii) a distal reference electrode located at a distal edge of the expandable distal-end assembly externally to the inner volume, and wherein the proximal reference ring electrode and the distal reference electrode are to be coupled with one another and for, respectively, (i) generating an AC signal between the reference electrodes, and (ii) sensing a resulting AC signal on each of the plurality of functional electrodes and a given electrode.
generating the AC signal between the distal reference electrode and the proximal reference ring electrode;
sensing the resulting electrical AC signal between each of the plurality of functional electrodes and the given electrode; and
based on the sensed AC signals, determining, for at least one given functional electrode from among the plurality of functional electrodes, a proximity of the functional electrode to wall tissue of the cardiac chamber.

### Example 12

The method according to example 11, wherein the given electrode is one of the proximal reference ring electrode, the distal reference electrode, a far-field electrode located within the inner volume, and another functional electrode on the spline.

### Example 13

The method according to example 11, wherein generating the AC signal comprises generating an AC voltage of constant magnitude, and wherein sensing the respectively resulting AC signal comprises sensing a resulting AC current.

### Example 14

The method according to example 11, wherein generating the AC signal comprises generating an AC current of constant magnitude and wherein sensing the respectively resulting AC signal comprises sensing a resulting AC voltage.

### Example 15

The method according to example 11, wherein the proximal reference ring electrode is located on a proximal base section of the expandable distal-end assembly externally to the inner volume.

### Example 16

The method according to example 11, wherein the distal-end assembly is a basket having an expandable frame comprising multiple splines that are electrically insulated from a surrounding environment, wherein the functional electrodes are coupled to the splines.

### Example 17

The method according to example 16, wherein the distal reference electrode is formed from distal ends of the splines that are (i) electrically exposed to the surrounding environment and (ii) electrically connected to one another.

### Example 18

The method according to example 11, wherein the distal-end assembly is a balloon assembly having an expandable membrane, and wherein the functional electrodes are disposed over the membrane.

### Example 19

The method according to example 18, wherein the distal reference electrode is disposed over a distal end of the membrane.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system (10), comprising:
a catheter (14), comprising:
a shaft (44) having a distal end (46) configured for insertion into a cardiac chamber of a patient (23); and
an expandable distal-end assembly (28) that when expanded defines an inner volume (77), the distal-end assembly comprising:
a plurality of functional electrodes (26) that are at least partially external to the inner volume (77) and are configured to be placed in contact with wall tissue (131) of the cardiac chamber;
a proximal reference electrode (17) located at a proximal end of the expandable distal-end assembly (28) externally to the inner volume (77) ;
a distal reference electrode (37) located at a distal edge (16) of the expandable distal-end assembly (28) externally to the inner volume (77); and
wherein the proximal reference ring electrode (17) and the distal reference electrode (37) are to be coupled with one another and for, respectively, (i) generating an AC signal between the reference electrodes (17, 37), and (ii) sensing a resulting AC signal on each of the plurality of functional electrodes (26) and a given electrode (17, 37, 223);
a signal generator (35) configured to generate the AC signal between the distal reference electrode (37) and the proximal reference ring electrode (17);
an interface (30) configured to sense the resulting AC signal between each of the plurality of functional electrodes (26) and the given electrode (17, 37, 223); and
a processor (56), which is configured to, based on the sensed AC signals, determine, for at least one given functional electrode (26) from among the plurality of functional electrodes, a proximity of the functional electrode (26) to wall tissue (131) of the cardiac chamber.

2. The system (10) according to claim 1, wherein the given electrode (26) is one of the proximal reference ring electrode (17, 217), the distal reference electrode (37, 237), a far-field electrode (223) located within the inner volume (77, 277), and another functional electrode (26) on the spline.

3. The system (10) according to claim 1 or claim 2, wherein the signal generator (35) is configured to generate the AC signal by generating an AC voltage of constant magnitude, and wherein the interface (30) is configured to sense the respectively resulting AC signal by sensing a resulting AC current.

4. The system (10) according to claim 1 or claim 2, wherein the signal generator (35) is configured to generate the AC signal by generating an AC current of constant magnitude, and wherein the interface (30) is configured to sense the respectively resulting AC signal by sensing a resulting AC voltage.

5. The system (10) according to any preceding claim, wherein the proximal reference ring electrode (17, 217) is located on a proximal base (227) section of the expandable distal-end assembly (28, 281) externally to the inner volume (77, 277).

6. The system (10) according to any preceding claim, wherein the distal-end assembly (28, 281) is a basket having an expandable frame comprising multiple splines (22, 222) that are electrically insulated from a surrounding environment, wherein the functional electrodes (26, 226) are coupled to the splines (22, 222).

7. The system (10) according to claim 6, wherein the distal reference electrode (37, 237) is formed from distal ends of the splines (22, 222) that are (i) electrically exposed to the surrounding environment and (ii) electrically connected (257) to one another.

8. The system (10) according to claim 1, wherein the distal-end assembly is a balloon assembly having an expandable membrane, and wherein the functional electrodes are disposed over the membrane.

9. The system (10) according to claim 8, wherein the distal reference electrode is disposed over a distal end of the membrane.
